# DEMANDE DE BREVET EUROPEEN

(11) **EP 0 795 314 A1**
(43) Date de publication de la demande: **17.09.1997**
(21) Numéro de dépôt: 97400538.1
(22) Date de dépôt: 11.03.1997
(51) Int. Cl.: A61K 7/155

(54) **Composition épilatoire comprenant des polymères de latex et des résines et procédé d'épilation**

(30) Priorité: 15.03.1996 FR 9603307
(71) Demandeur: SEB S.A., 69130 Ecully (FR)
(72) Inventeur: Paget, Monique, 69008 Lyon (FR); Bontoux Daniel, 69230 Saint-Genis-Laval (FR); Boccaccio, Guy, 72000 Le Mans (FR); Legeais, Véronique, 72000 Le Mans (FR)
(74) Mandataire: Phélip, Bruno

(57) **Abrégé**

Composition épilatoire comprenant:
- au moins un polymère de latex, et
- au moins une résine tackifiante compatible avec ledit polymère.

Une couche de la dite composition est appliquée sur la zone du corps à épiler, puis séchée à une température permettant sa prise en masse et la formation d'un film, qui est alors pelé.

## Description

La présente invention a pour objet une composition épilatoire comprenant des polymères de latex et des résines.

Elle est en outre relative à un procédé d'épilation utilisant cette composition.

Plusieurs types de compositions épilatoires sont déjà connus.

Les compositions à base de cires, les plus répandues, sont appliquées sous forme fondue sur les surfaces à épiler, puis une fois durcies sont arrachées avec les poils qui y sont physiquement emprisonnés. L'inconvénient majeur de ce type de composition est la nécessité de faire fondre les compositions dans un récipient avant de les appliquer sur la peau. Les temps de fusion des masses de cire sont assez longs, de l'ordre de 18 minutes, et entraînent une perte de temps pour l'utilisateur. En outre la chaleur des cires fondues peut incommoder certains utilisateurs.

De plus, certaines de ces compositions une fois durcies présentent une faible résistance, et il est donc nécessaire de les appliquer en couche épaisse, ce qui n'est pas sans poser de problème, du fait de la difficulté à appliquer les cires en épaisseur homogène.

On connaît aussi des compositions épilatoires à base de sucres, contenant de faibles quantités de cires. Ces compositions nécessitent aussi un chauffage modéré. De plus, du fait de la faible résistance du film durci formé sur la peau, l'utilisation d'un film-support tel qu'un morceau de tissu, sur lequel cette composition est étalée, est nécessaire.

D'autres compositions épilatoires comprenant un mélange de silicones ont été décrites. Néanmoins, ces compositions ont un temps de prise très important et nécessitent une réaction chimique, qui peut être une source de désagréments pour l'utilisateur.

Des adhésifs épilatoires comprenant un mélange d'élastomère thermoplastique, d'une résine collante, d'une gomme butylique et d'un polymère liquide soluble dans les trois premiers composants, ont déjà été décrits dans le brevet JP 54 91 908.

Un autre document illustratif de l'état de la technique est le brevet US 4 282 877 qui décrit un produit épilatoire comprenant obligatoirement un support, tel qu'une feuille de polyester, sur lequel se trouve la composition épilatoire à proprement parler. Après application sur la peau, le support peut être manipulé pour entraîner les poils collés à la substance épilatoire. Un autre produit épilatoire de ce type est décrit dans l'abrégé du brevet japonais JP 58 160 381. Le support comprend une composition thermofusible d'élastomères et de résines tackifiantes.

Le brevet européen EP-299 816 décrit une composition comprenant 5 à 15% en poids d'un composé macromoléculaire élastomère et/ou thermoplastique, 40 à 80% d'une colophane naturelle et/ou une colophane modifiée, et 10 à 20% d'un assouplissant, tel que notamment une graisse, une huile ou une cire. Selon le mode de mise en oeuvre préférentiel décrit dans ce document, la composition épilatoire est préalablement chauffée, puis est appliquée à l'aide d'une spatule sur les parties à épiler. Après prise en masse de cette composition, la pellicule plastique formée qui emprisonne les poils est pelée.

Selon ce brevet, il serait possible, en remplaçant dans la formule les colophanes par "certains types de colophanes ou de résines liquides", d'utiliser le produit à épiler à température ambiante, sans avoir besoin de le chauffer préalablement. Cependant, aucune précision n'est donnée quant aux colophanes ou aux résines liquides qui se substitueraient à celles indiquées dans le brevet. En outre, ce brevet ne mentionne pas de traitement de la composition ainsi appliquée à température ambiante. Il faut donc supposer que le produit sèche, sans traitement particulier, et forme un film. Or, il s'avère que l'enseignement divulgué dans ce brevet ne permet pas d'obtenir un film et d'effectuer des épilations. En conséquence, le contenu de ce document est non seulement incertain, car les composés rentrant dans la composition des formules indiquées ne sont pas décrits, mais de plus insuffisant car il ne permet pas d'obtenir le résultat prévu, à savoir l'épilation.

Il ressort de l'état de la technique analysé ci-dessus que les compositions épilatoires déjà connues présentent des inconvénients les rendant d'une utilisation peu pratique ou se révèlent peu efficaces.

Le demandeur a résolu ces problèmes en trouvant une composition liquide pouvant être appliquée sur les parties du corps à épiler à température ambiante et prenant rapidement en masse par séchage à une température modérée, tout en formant un film suffisamment résistant pour pouvoir être pelé sans qu'il soit nécessaire de l'étaler au préalable sur un support.

La présente invention a donc pour objet une composition épilatoire durcissant par séchage, ne nécessitant pas de support et comprenant:
- au moins un polymère de latex, et
- au moins une résine exerçant une fonction d'adhésion, dite " tackifiante " compatible avec ledit polymère.

On entend par résine " tackifiante" une résine présentant un pouvoir collant suffisant pour permettre l'arrachage des poils.

Ledit polymère de latex peut être d'origine naturelle ou d'origine synthétique. Il peut être notamment un copolymère d'acétate de vinyle et d'ester maléique, un copolymère de styrène butadiène carboxylé, ou un mélange de ces deux polymères. Néanmoins, tout autre polymère de latex peut convenir, pour autant qu'il présente une compatibilité chimique et physico-chimique satisfaisante avec la résine et les autres composants éventuels.

Ledit polymère peut être réticulé, avant d'être introduit dans la composition, afin d'augmenter sa rigidité. De manière avantageuse, une telle pré-réticulation peut être effectuée par traitement du polymère avec de l'oxyde de zinc ou avec de l'aziridène trifonctionnel. La réticulation par l'oxyde de zinc est effectuée en ajoutant jusqu'à 15% en poids en particulier environ 10% en poids, par rapport au polymère sec, de cet oxyde, soit sous forme de poudre, soit sous forme de dispersion aqueuse et en portant le mélange réactionnel à une température ambiante ou à 60°C, durant entre 24 et 48 heures.

La réticulation par l'aziridène trifonctionnel est réalisée par mise en contact du polymère avec une préparation à base d'aziridine, par exemple celle commercialisée par la Société ICI sous la dénomination Néocryl CX-100 à température ambiante durant 24 heures ou à 60°C durant 2 heures. En raison du caractère irritant de ce produit, les compositions selon la présente invention n'en contiennent préférentiellement pas plus de 2% en poids. La détermination de la réticulation optimale peut être effectuée à l'aide de rayonnement infrarouge ou par chromatographie en phase gazeuse (CPG).

Avantageusement, la composition selon l'invention contient entre 70 et 97%, préférentiellement de 80 à 93% et encore plus préférentiellement environ 90% de polymère.

La résine tackifiante rentrant dans la composition selon l'invention est avantageusement une colophane ou une résine terpénique. Elle peut néanmoins être toute autre résine, ou tout autre composé présentant des propriétés comparables aux résines.

Une résine colophane particulièrement appropriée pour la mise en oeuvre de la présente invention est la résine MBG 166 commercialisée par HERCULES BV. Une résine terpénique peut être celle commercialisée sous la référence DERMULSENE DT 50 par la Société DRT.

Avantageusement, la composition selon l'invention contient entre 3 et 30%, préférentiellement 7 à 20% et encore plus préférentiellement environ 10% en poids de résine tackifiante.

Bien que des compositions contenant uniquement des polymères et des résines permettent d'obtenir des compositions épilatoires efficaces, d'autres composés peuvent être ajoutés. De tels composés peuvent être ajoutés. De tels composés peuvent être des principes actifs en phase aqueuse, tels que des produits empêchant la repousse des poils, des produits apaisants, des colorants ou des parfums.

Des compositions particulièrement avantageuses entrant dans le cadre de la présente invention sont les suivantes, les pourcentages étant exprimés en poids.
Composition (a):
   70% de copolymère d'acétate de vinyle et d'ester maléique,
   20% de copolymère de styrène butadiène carboxylé,
   10% de colophane.
Composition (b):
   90% de copolymère de styrène butadiène carboxylé, réticulé par de l'oxyde de zinc, et
   10% de colophane,
Composition (c):
   90% de copolymère de styrène butadiène carboxylé, réticulé par de l'aziridène trifonctionnel, et
   10% de colophane.

La composition selon la présente invention peut être préparée par mélange des polymères et des résines, et éventuellement des autres composés, par des méthodes connues de l'homme du métier.

La présente invention a d'autre part pour objet un procédé d'épilation d'une zone du corps à épiler comprenant les étapes suivantes:
- application sur ladite zone d'une couche d'une composition selon l'invention, telle que décrite ci-dessus,
- séchage de ladite couche à une température permettant sa prise en masse et la formation d'un film, et
- pelage du film sec.

L'application de la composition peut être effectuée par étalement à l'aide d'une spatule ou d'un rouleau. L'épaisseur de la couche appliquée est préférentiellement comprise entre 0,2 mm et 0,7 mm.

Le séchage du film est avantageusement effectué par traitement de la couche de composition à l'aide d'un courant d'air chaud, de préférence sec, préférentiellement à une température d'au moins 40°C et encore plus préférentiellement d'au moins 50°C. De telles conditions sont remplies, par exemple lors du séchage à l'aide d'un sèche cheveux réglé de manière appropriée.

La composition selon l'invention et son procédé d'application présentent un grand nombre d'avantages par rapport aux compositions et aux procédés décrits dans l'état de la technique. La composition selon l'invention peut être étalée sur la peau, à température ambiante, c'est-à-dire sans chauffage préalable. Le durcissement du film est obtenu à température modérée pouvant être mis en oeuvre simplement à l'aide d'air chaud, sans intervention de réactifs chimiques. Le procédé ne nécessite aucun appareillage particulier. Enfin, du fait de la résistance du film formé par séchage de la composition, l'utilisation d'un support, tel qu'une feuille de polyester, n'est pas nécessaire.

La présente invention est illustrée sans pour autant être limitée par les exemples suivants:

### EXEMPLE 1:

### Préparation d'une composition contenant un copolymère d'acétate de vinyle et d'ester maléique, un copolymère de styrène butadiène carboxylé, et une résine colophane.

67,5 parties en poids de Rhodopas AM 021 (Rhône Poulenc), dispersion aqueuse non ionique d'un copolymère d'acétate de vinyle et d'ester maléique, ont été mélangés par agitation magnétique avec 10 parties en poids de MBG 166, ou TACOLYN 3166, colophane commercialisée par HERCULES BV.

22,5 parties en poids de Rhodopas SB 722, dispersion aqueuse anionique de copolymère de styrène butadiène carboxylé, sont ensuite dispersés dans le mélange.

Cette composition qui présente initialement un extrait sec de 54% a été concentrée par évaporation lente sous agitation, jusqu'à atteindre un extrait sec de 60%, conférant à la composition une consistance de crème cosmétique.

La concentration est effectuée dans un réacteur ouvert, muni d'une ancre d'agitation et d'une double enveloppe thermostatée à 40°C.

On a étudié l'influence des conditions opératoires sur la durée de séchage des films et les propriétés mécaniques des films secs. Les résultats figurent dans le tableau I.

### EXEMPLE 2:

### Préparation d'une composition épilatoire contenant un copolymère de styrène butadiène carboxylé réticulé par de l'oxyde de zinc et une résine colophane.

15 parties en poids d'oxyde de zinc sont ajoutées à 85 parties en poids de Rhodopas SB 722 sous agitation et la réaction est effectuée à température ambiante durant une heure.

Le mélange est alors porté à 60°C pendant 24 heures, dans un bain-marie, puis après retour à la température ambiante, le mélange est filtré sur une toile de nylon pour éliminer d'éventuels coagulats.

On mélange 90 parties en poids de Rhodopas SB 722 ainsi réticulée, dans les conditions décrites dans l'exemple 1, à 10 parties en poids de MBG 166.

Les propriétés de cette composition qui présentent un extrait sec de 58% figurent dans les tableaux I et Il ainsi que celles de la composition concentrée jusqu'à un extrait sec de 60%.

### EXEMPLE 3:

### Obtention d'une composition épilatoire contenant un copolymère de styrène butadiène carboxylé et réticulé par de l'aziridène trifonctionnel et une résine colophane.

On fait réagir 1 partie en poids d'aziridène trifonctionnel dilué à 50% commercialisé sous la dénomination Crosinker CX-100 par ICI avec 99 parties en poids de Rhodopas SB 722.

Le mélange est maintenu sous agitation à 60°C pendant 2 heures, puis après retour à la température ambiante, filtré sur une toile de nylon.

90 parties en poids de Rhodopas 722 ainsi réticulé sont mélangées dans les conditions indiquées dans l'exemple 1 avec 10 parties en poids de MBG 166.

Les propriétés de cette composition, ayant un extrait sec de 51%, ainsi que celles de la même composition évaporée sous agitation présentant un extrait sec de 60%, figurent dans les tableaux I et II.

### EXEMPLE 4:

### Influence des conditions de séchage sur les propriétés mécaniques des films secs.

On a étalé une épaisseur de 0,4 mm des compositions des exemples 1, 2 et 3 laissées à température ambiante sur une plaque de verre chauffée à 35°C, qui est approximativement la température de la peau.

Deux types de séchage avec sèche-cheveux ont été testés: en atmosphère sèche et en atmosphère humide.

Le séchage en atmosphère humide a été réalisé par apport d'un nuage de vapeur d'eau entre le sèche-cheveux et la couche de composition, en contrôlant la température en surface de manière qu'elle ne dépasse pas 50-55°C.

II ressort de l'analyse des tableaux I et Il que, de manière générale, les films secs obtenus présentent une épaisseur comprise entre 0,20 et 0,25 mm et des propriétés mécaniques satisfaisantes.

### EXEMPLE 5:

### Essai d'épilation sur la peau.

Les compositions des exemples 1 à 3 présentant 60% d'extrait sec ont été étalées à l'aide d'une spatule sur une surface de peau de la jambe d'environ 3 x 5 cm² avec une épaisseur sensiblement régulière d'environ 0,5 mm.

Elles ont été ensuite séchées au sèche cheveux à 50°C durant 7 minutes (exemple 1) ou 5 minutes (exemples 2 et 3).

Le film qui présente une épaisseur d'environ 0,25 à 0,30 mm se pèle rapidement et présente une bonne cohésion.

Les résultats d'épilation ont été jugés très satisfaisants par l'utilisatrice par comparaison aux compositions classiques à base de cire.

L'utilisatrice a noté que l'utilisation des compositions selon la présente invention n'induisait pas de choc thermique ni de sensation de forte chaleur sur la peau, comme ceci est le cas des cires appliquées à chaud. Les pelages des films ont été jugés comparables dans les deux cas. La surface de la peau traitée est correctement épilée avec les compositions selon la présente invention.Les compositions selon la présente invention n'induisent pas non plus de résidus difficiles à éliminer.

En outre, il n'y a pas eu d'apparition de rougeur sur la peau.

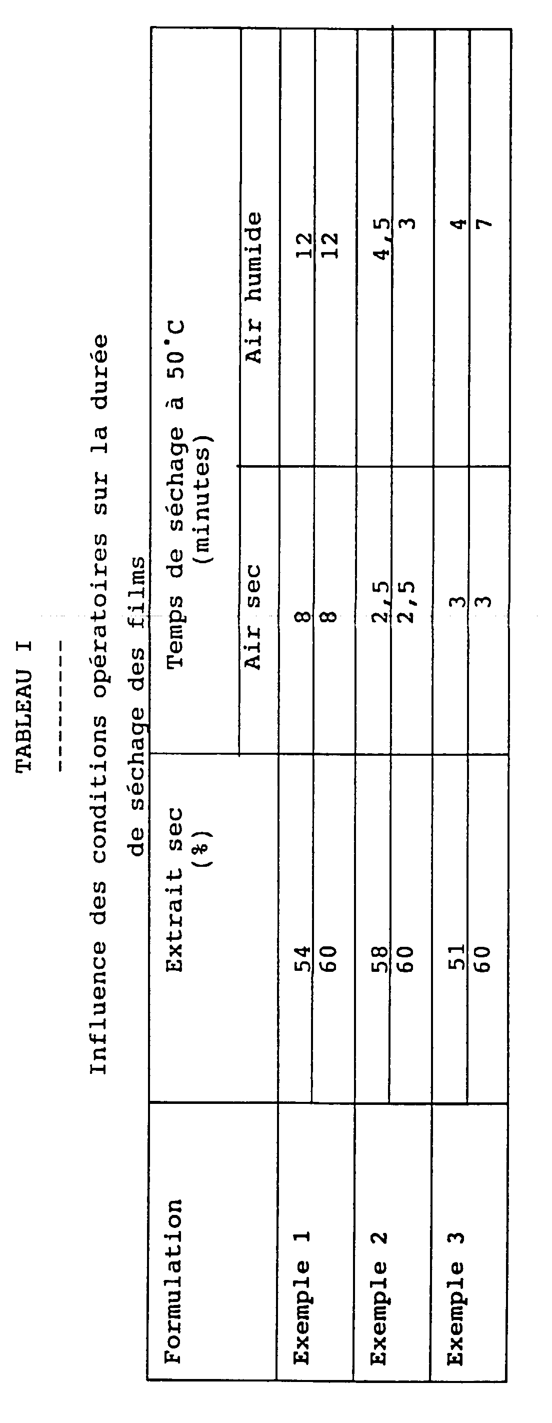

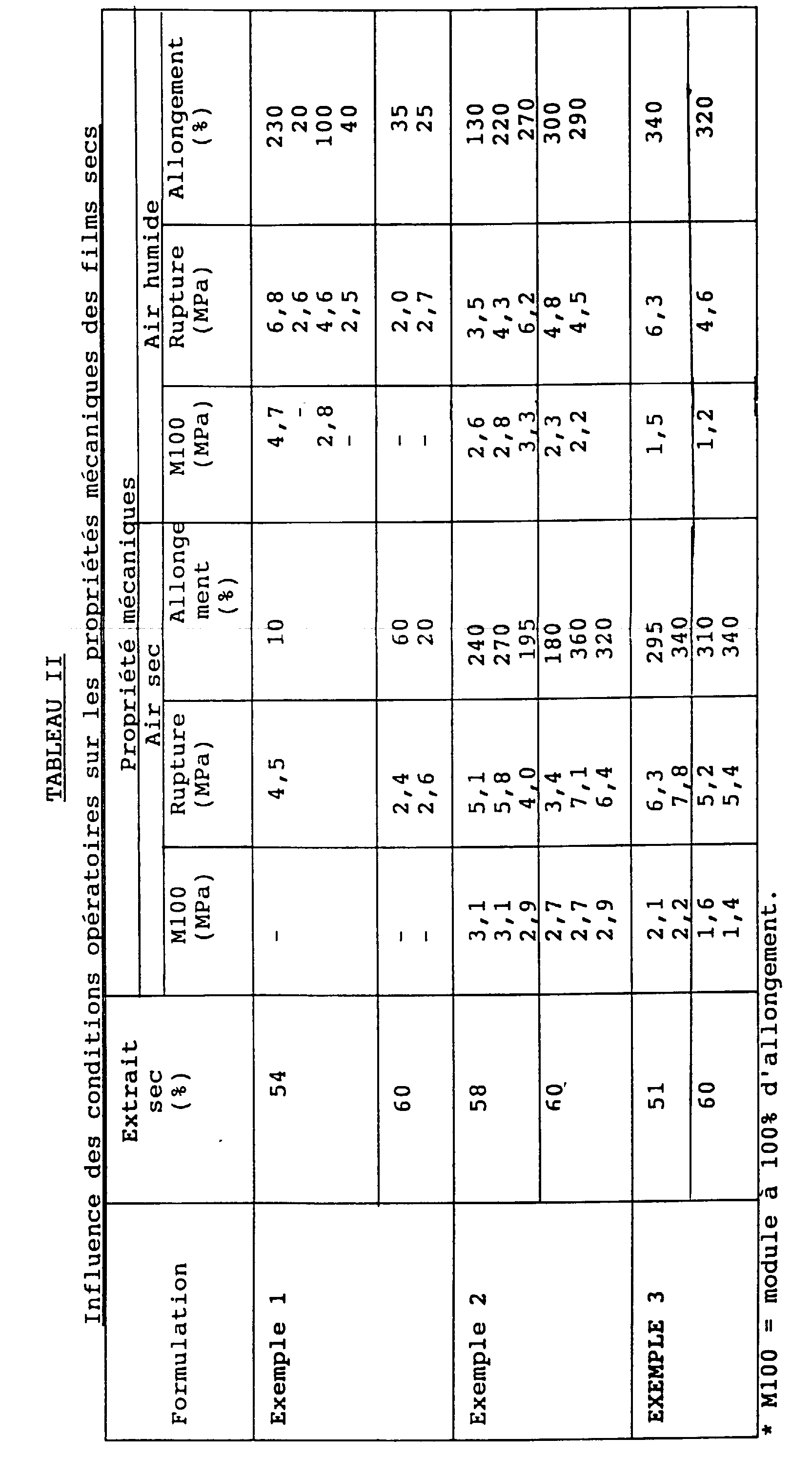

## Revendications

1. Composition épilatoire durcissant par séchage, ne nécessitant pas de support et comprenant:
- au moins un polymère de latex, et
- au moins une résine tackifiante compatible avec ledit polymère.

2. Composition selon la revendication 1 caractérisée en ce que ledit polymère est un copolymère d'acétate de vinyle et d'ester maléique, un copolymère de styrène-butadiène carboxylé, ou un mélange de ces deux copolymères.

3. Composition selon l'une des revendications 1 et 2 caractérisée en ce que ledit polymère est réticulé.

4. Composition selon la revendication 3 caractérisée en ce que la réticulation est effectuée par traitement du polymère par de l'oxyde de zinc ou de l'aziridène trifonctionnel.

5. Composition selon l'une des revendications 1 à 4 caractérisée en ce qu'elle contient en poids de 70 à 97%, préférentiellement de 80 à 93% et encore plus préférentiellement environ 90% de polymère.

6. Composition selon l'une des revendications 1 à 5 caractérisée en ce que ladite résine est une colophane ou une résine terpénique.

7. Composition selon l'une des revendications 1 à 6 caractérisée en ce qu'elle contient en poids de 3 à 30%, préférentiellement de 7 à 20% et encore plus préférentiellement environ 10% de résine.

8. Composition selon l'une des revendications 1 à 7 caractérisée en ce qu'elle contient environ :
- 70% de copolymère d'acétate de vinyle et d'ester maléique,
- 20% de copolymère de styrène butadiène carboxylé, et
- 10% de colophane.

9. Composition selon l'une des revendications 1 à 7 caractérisée en ce qu'elle contient environ:
- 90% de copolymère de styrène butadiène carboxylé, réticulé par de l'oxyde de zinc, et
- 10% de colophane.

10. Composition selon l'une des revendications 1 à 7 caractérisée en ce qu'elle contient environ:
- 90% de copolymère de styrène butadiène carboxylé réticulé par de l'aziridène trifonctionnel, et
- 10% de colophane.

11. Procédé d'épilation d'une zone du corps à épiler comprenant les étapes suivantes:
- application sur ladite zone d'une couche de composition selon l'une des revendications 1 à 10,
- séchage de ladite couche à une température permettant sa prise en masse et la formation d'un film, et
- pelage du film sec.

12. Procédé selon la revendication 11 caractérisé en ce que le séchage est effectué par traitement de la couche de composition à l'aide d'un courant d'air chaud, par exemple à l'aide d'un sèche-cheveux.

13. Procédé selon l'une des revendications 11 et 12 caractérisé en ce que la température de séchage est préférentiellement d'au moins 40°C et encore plus préférentiellement d'au moins 50°C.

14. Procédé selon l'une des revendications 11 à 13 caractérisé en ce que l'épaisseur de la couche appliquée sur la peau est comprise entre environ 0,2 mm et 0,7 mm.
